# EUROPEAN PATENT APPLICATION

(11) **EP 3 150 232 A1**
(43) Date of publication of application: **05.04.2017**
(21) Application number: 14892931.8
(22) Date of filing: 10.06.2014
(51) Int. Cl.: A61K 49/18, A61K 49/08

(54) **RARE EARTH-BASED NANOPARTICLE MAGNETIC RESONANCE CONTRAST AGENT AND PREPARATION METHOD THEREOF**

(30) Priority: 29.05.2014 CN 201410233943
(71) Applicant: Peking University, Beijing 100871 (CN)
(72) Inventor: SUN, Lingdong, Beijing 100871 (CN); YAN, Chunhua, Beijing 100871 (CN); ZHENG, Xiaoyu, Beijing 100871 (CN)
(74) Representative: Brown, David Leslie
(86) International application number: PCT/CN2014/079597
(87) International publication number: WO 2015/180205

(57) **Abstract**

The present invention relates to a rare earth-based nanoparticle magnetic resonance contrast agent and a preparation method thereof. The rare earth-based nanoparticle magnetic resonance contrast agent is rare earth-based inorganic nanoparticles having the surfaces coated with hydrophilic ligands. The rare earth-based nanoparticles are first obtained by a high-temperature oil phase reaction, and then the surfaces thereof are coated with hydrophilic molecules to obtain the rare earth-based nanoparticle magnetic resonance contrast agent. Compared with the existing clinical contrast agent, the magnetic resonance contrast agent of the present invention has a greatly improved relaxivity, a good imaging effect, a low required injection dose, and long in vivo residence time. In addition, the rigid structure of the inorganic nanoparticles can effectively reduce the leakage possibility of gadolinium ions.

## Description

### Technical Field

The present invention relates to a rare earth-based nanoparticle magnetic resonance contrast agent and a preparation method thereof, and belongs to the technical field of nano materials.

### Background Art

Magnetic Resonance Imaging (MRI) is an important technique in the medical diagnosis and molecular imaging field, and has such advantages as high tissue resolution, multiple imaging parameters and no radiation damage to human bodies. However, as the MRI technology has a low sensitivity, contrast agents are often employed to improve the imaging contrast ratio and the image quality clinically. According to the ratio of the transverse relaxivity to the longitudinal relaxivity, contrast agents can be divided into two categories: T₁ contrast agents brightening local tissues and T₂ contrast agents darkening local tissues. With unfilled 4f electronic shells, rare earth ions possess unique optical, electrical and magnetic properties, and thus have important application value in both aspects of magnetic resonance T₁ and T₂ contrast agents.

In the aspect of T₁ contrast agents, trivalent gadolinium ions (Gd³⁺) have the largest number of unpaired electrons, and a long electron spin relaxation time, which can effectively shorten the longitudinal relaxation time to increase the image lightness, and are thus regarded as the best choice of the T₁ contrast agents. In order to reduce the toxicity risk that the free gadolinium ions bring about, currently mostly widely-used T₁ contrast agents are gadolinium-containing paramagnetic chelates, to reduce the leakage possibility by a chelating mode. However, such contrast agents typically have a low relaxivity, limited contrasting effect, and a large required dose, and still have potential threats for normal tissues. In addition, as such contrast agents belong to a small molecule and have a short in vivo residence time, the diagnostic effect over a long time cannot be guaranteed.

In the aspect of T₂ contrast agents, superparamagnetic iron oxide nanoparticles as contrast agents have been commercialized, but unfortunately such contrast agents will reach a saturated magnetization at a relatively low magnetic field strength (1.5 T), and therefore the contrasting effect is poor at a higher magnetic field strength (NaDyF4 Nanoparticles as T-2 Contrast Agents for Ultrahigh Field Magnetic Resonance Imaging, Frank C. J. M. van Veggel, et al. J. Phys. Chem. Lett. 2012, 3, 524-529). However the rare earth ions (such as terbium Tb³⁺, dysprosium Dy³⁺, holmium Ho³⁺, erbium Er³⁺) have a large magnetic moment and a short electron spin relaxation time; therefore, they are expected to meet the requirements of contrasting at a high magnetic field strength.

In summary, rare earth-based nanoparticles are expected to become a new generation of highly efficient magnetic resonance contrast agents, because individual particles contain a large amount of rare earth ions, and can produce a more significant signal enhancement, and the rigid skeleton of inorganic nano structures can reduce the leakage possibility of the rare earth ions. Moreover, as the sizes of nanoparticles are greater than those of chelates, the in vivo circulation time is relatively long. In addition, the surfaces of inorganic nano structures can be easily modified with functional groups to achieve the purposes of active targeting, and multi-mode imaging and so on. Therefore, the development and utilization of the rare earth-based nanoparticle magnetic resonance contrast agent has a considerable significance for improving diagnostic accuracy and safety of the contrast agent.

### Summary of the Invention

The present invention provides a rare earth-based nanoparticle magnetic resonance contrast agent and a preparation method thereof, and the magnetic resonance contrast agent has such advantages as high relaxivity, small injection dose, long in vivo circulation time, and low leakage possibility of the rare earth ions.

The rare earth-based nanoparticle magnetic resonance contrast agent of the present invention refers to rare earth-based inorganic nanoparticles with the surfaces thereof coated with hydrophilic ligands. In the present invention, the rare earth-based nanoparticles are first obtained by a high-temperature oil phase reaction, and then the surfaces thereof are coated with hydrophilic molecules to obtain the rare earth-based nanoparticle magnetic resonance contrast agent.

Rare earth elements (RE) in the rare earth-based nanoparticle magnetic resonance contrast agent of the present invention comprise one or more of lanthanum (La), cerium (Ce), praseodymium (Pr), neodymium (Nd), samarium (Sm), europium (Eu), gadolinium (Gd), terbium (Tb), dysprosium (Dy), holmium (Ho), erbium (Er), thulium (Tm), ytterbium (Yb), lutetium (Lu), scandium (Sc), and yttrium (Y).

The composition of the rare earth-based nanoparticles in the rare earth-based nanoparticle magnetic resonance contrast agent of the present invention is MₐREO_{b}X_{c}, wherein RE represents a rare earth element, M represents an alkali or alkaline earth metal, X represents a fluorine or chlorine, 0≤a≤1, 0≤b≤1.5, and 0≤c≤4. In addition, the rare earth-based nanoparticles can also be an inorganic compound doped by using MₐREO_{b}X_{c} as a substrate, and the doping serves to impart them a luminescent property or control their magnetic property.

The surface coating ligands of the rare earth-based nanoparticle magnetic resonance contrast agent of the present invention can employ one or more of the following: a small hydrophilic molecule such as citric acid and cysteine, and a hydrophilic polymer such as a polyvinyl alcohol, polyethyleneimine, polyvinyl pyrrolidone, and polyacrylic acid.

The present invention provides a preparation method of a rare earth-based nanoparticle magnetic resonance contrast agent, wherein the method comprises the following steps:
1) adding a certain amount of a rare earth precursor or a mixture of a rare earth precursor and a non-rare earth precursor into a high-boiling organic solvent to obtain a solution A;
2) performing vacuum pumping on the solution A to remove moisture, then heating up to 250-340°C under the protection of an inert gas and maintaining for 15 min-24 h, and then cooling to room temperature to obtain a sol B;
3) performing centrifugal separation on the sol B, washing the obtained precipitate, and then coating the surface of the precipitate with hydrophilic ligands;
4) dispersing the coated particles into a solvent to obtain the contrast agent.

In step 1), the molar ratio of the precursor to the solvent is preferably 1:20-1:200, the rare earth precursor in the precursor must be added, and whether the non-rare earth precursor needs to be added depends on the composition of a target product; in step 2), vacuum pumping is performed preferably at 100-140°C; in step 3), a large amount of ethanol is preferably employed to wash, a washing manner is preferably centrifugal washing, and washing is preferred for 2 to 6 times; and in step 4), the solvent is preferably water or physiological saline.

The high-boiling organic solvent in the present invention refers to a mixed solvent composed of one or more of oleic acid, linoleic acid, oleylamine, octadecene, hexadecylamine and octadecylamine.

The rare earth precursor in the present invention is a mixture of one or more of the following: rare-earth hydroxides, oxalates, acetates, trifluoroacetates, trichloroacetates, acetylacetonates, and phenyl acetylacetonates.

The non-rare earth precursor in the present invention is a mixture of one or more of the following: alkali-metal and alkaline earth-metal fluorides, hydroxides, oxalates, acetates, trifluoroacetates, trichloroacetates, acetylacetonates, and phenyl acetylacetonates.

In the preparation method of the rare earth-based nanoparticle magnetic resonance contrast agent of the present invention, the composition, size, shape and crystallization of the rare earth-based nanoparticles can be adjusted by adjusting the parameters of the solvent ratio, the feeding amount of the precursor, the reaction temperature, the reaction time, and the like; and the relaxation property, the biocompatibility and the like of the contrast agent can be adjusted by the parameters of the type, the feeding amount and the like of water-soluble molecules during the surface coating of the hydrophilic ligands.

The rare earth-based nanoparticle magnetic resonance contrast agent of the present invention has the following advantages:
1. the individual particles of the magnetic resonance contrast agent of the present invention contain a large number of rare earth ions, which can significantly reduce the relaxation time of surrounding protons;
2. the magnetic resonance contrast agent of the present invention has a larger size than chelates, and a long in vivo circulation time, which can meet the requirement of a long time clinical diagnosis;
3. the magnetic resonance contrast agent of the present invention has a relatively high relaxivity, which can be about ten times higher than that of the clinically commonly-used contrast agent, and therefore provides a better contrasting effect under the condition of the same concentration;
4. the magnetic resonance contrast agent of the present invention has a rigid skeleton of an inorganic nano structure, which can reduce the leakage possibility of rare earth ions, and therefore is safer compared with chelates;
5. since the magnetic resonance contrast agent of the present invention features an excellent imaging performance, the required dose can be greatly reduced compared with the currently clinically commonly-used contrast agent, further reducing the safety risk;
6. the magnetic resonance contrast agent of the present invention features easy control, simple reaction operations, good repeatability, and stable properties.

### Brief Description of the Drawings

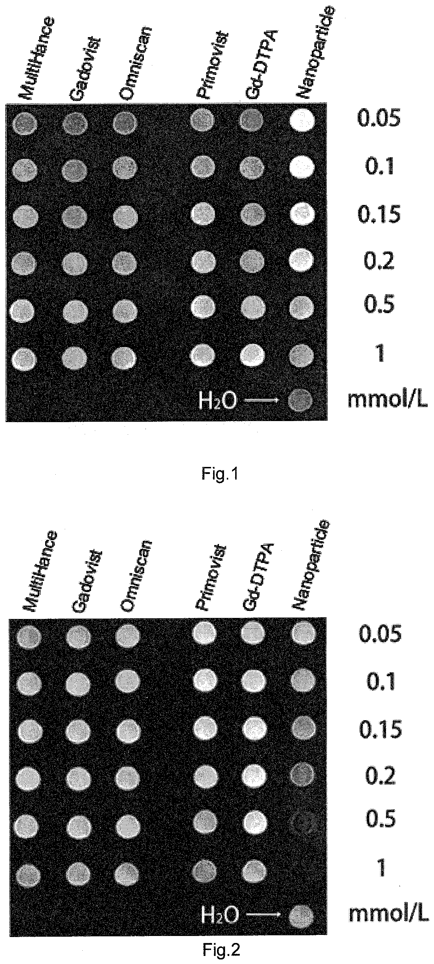

Fig. 1 shows a contrast of magnetic resonance images obtained by using a rare earth-based nanoparticle magnetic resonance contrast agent and five clinically commonly-used contrast agents under different concentrations, wherein the used scanning sequence is a T₁ weighted sequence, and the used magnetic field strength is 3 T.
Fig. 2 shows a contrast of magnetic resonance images obtained by using a rare earth-based nanoparticle magnetic resonance contrast agent and five clinically commonly-used contrast agents under different concentrations, wherein the used scanning sequence is a T₂ weighted sequence, and the used magnetic field strength is 3 T.

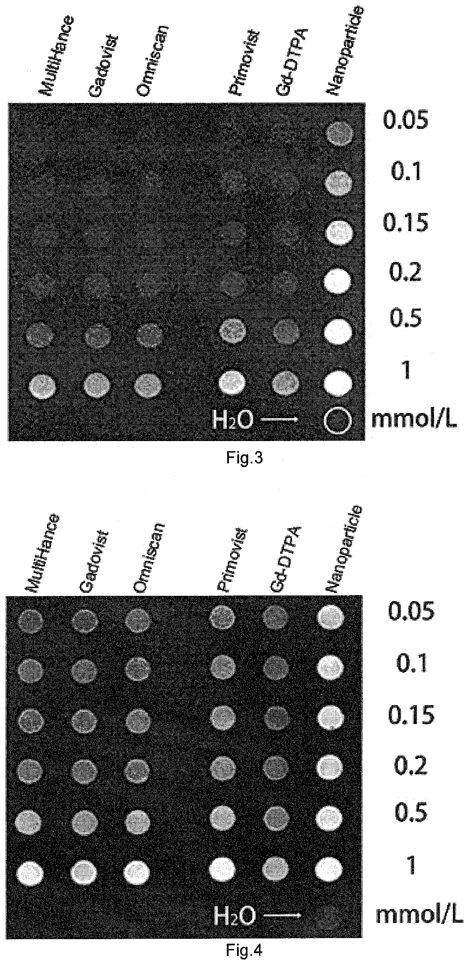

Fig. 3 shows a contrast of magnetic resonance images obtained by using a rare earth-based nanoparticle magnetic resonance contrast agent and five clinically commonly-used contrast agents under different concentrations, wherein the used scanning sequence is a ceMRA sequence, and the used magnetic field strength is 3 T.
Fig. 4 shows a contrast of magnetic resonance images obtained by using a rare earth-based nanoparticle magnetic resonance contrast agent and five clinically commonly-used contrast agents under different concentrations, wherein the used scanning sequence is a LAVA sequence, and the used magnetic field strength is 3 T.

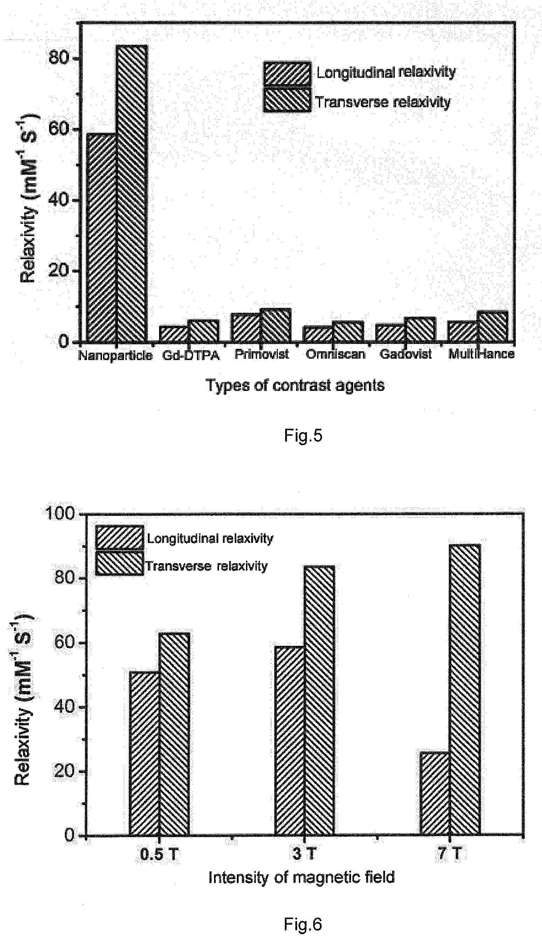

Fig. 5 is a diagram showing a contrast of relaxivities obtained by using a rare earth-based nanoparticle magnetic resonance contrast agent and five clinically commonly-used contrast agents, wherein the used magnetic field strength is 3 T.
Fig. 6 shows a contrast of relaxivities obtained by using a rare earth-based nanoparticle magnetic resonance contrast agent at different magnetic field strengths.

### Detailed Description of the Invention

The following describes the rare earth-based nanoparticle magnetic resonance contrast agent and the preparation method thereof of the present invention in connection with specific embodiments, so as to make the public better understand the technical contents, rather than to limit the technical contents. Actually, the improvements which are made for the composite material and the preparation method thereof with same or similar principles all fall within the protection scope of the present application. The following only takes a 50 ml capacity reaction system as an example to exemplify the embodiments, and the present invention can be implemented in a mode of same proportional amplification of each material in actual preparations.

### Embodiment 1

Synthesis of Gd₂O₃ nanoparticles: adding 0.5 mmol of gadolinium acetylacetonate into a mixed solvent of oleic acid (4 mL) and oleylamine (12 mL), heating up to 340°C under the protection of an inert gas, maintaining the temperature for 15 min, cooling the reaction solution to room temperature, adding a large amount of ethanol thereinto, and performing centrifugal washing twice to obtain the Gd₂O₃ nanoparticles.

### Embodiment 2

Synthesis of Pr₂O₃ nanoparticles: adding 0.5 mmol of praseodymium acetate into a mixed solvent of oleic acid (6 mL) and oleylamine (12 mL), heating up to 340°C under the protection of an inert gas, maintaining the temperature for 2 h, cooling the reaction solution to room temperature, adding a large amount of ethanol thereinto, and performing centrifugal washing twice to obtain the Pr₂O₃ nanoparticles.

### Embodiment 3

Synthesis of Er₂O₃ nanoparticles: adding 0.5 mmol of phenyl erbium acetylacetonate into a mixed solvent of oleic acid (6 mL) and oleylamine (8 mL), heating up to 310°C under the protection of an inert gas, maintaining the temperature for 1 h, cooling the reaction solution to room temperature, adding a large amount of ethanol thereinto, and performing centrifugal washing twice to obtain the Er₂O₃ nanoparticles.

### Embodiment 4

Synthesis of Y₂O₃ nanoparticles: adding 0.5 mmol of yttrium hydroxide into a mixed solvent of oleic acid (2 mL), oleylamine (3 mL), and octadecene (5 mL), heating up to 310°C under the protection of an inert gas, maintaining the temperature for 1 h, cooling the reaction solution to room temperature, adding a large amount of ethanol thereinto, and performing centrifugal washing twice to obtain the Y₂O₃ nanoparticles.

### Embodiment 5

Synthesis of LaF₃ nanoparticles: adding 1 mmol of lanthanum trifluoroacetate and 0.5 mmol of lithium fluoride into a mixed solvent of oleic acid (20 mmol) and octadecene (20 mmol), heating up to 260°C under the protection of an inert gas, maintaining the temperature for 4 h, cooling the reaction solution to room temperature, adding a large amount of ethanol thereinto, and performing centrifugal washing twice to obtain the LaF₃ nanoparticles.

### Embodiment 6

Synthesis of CeOF nanoparticles: adding 1 mmol of cerium oxalate into a mixed solvent of oleic acid (5 mmol) and hexadecylamine (35 mmol), heating up to 320°C under the protection of an inert gas, maintaining the temperature for 1 h, cooling the reaction solution to room temperature, adding a large amount of ethanol thereinto, and performing centrifugal washing twice to obtain the CeOF nanoparticles.

### Embodiment 7

Synthesis of EuOCl nanoparticles: adding 1 mmol of europium trichloroacetate into a mixed solvent of oleic acid (20 mmol) and octadecene (20 mmol), heating up to 330°C under the protection of an inert gas, maintaining the temperature for 1 h, cooling the reaction solution to room temperature, adding a large amount of ethanol thereinto, and performing centrifugal washing twice to obtain the EuOCl nanoparticles.

### Embodiment 8

Synthesis of NaDyF₄:Yb,Er nanoparticles: adding 0.78 mmol of dysprosium trifluoroacetate, 0.20 mmol of yttrium trifluoroacetate, 0.02 mmol of erbium trifluoroacetate, and 1 mmol of sodium trifluoroacetate into a mixed solvent of oleic acid (10 mmol), octadecylamine (10 mmol), and octadecene (20 mmol), heating up to 250°C under the protection of an inert gas, maintaining the temperature for 0.5 h, cooling the reaction solution to room temperature, adding a large amount of ethanol thereinto, and performing centrifugal washing four times to obtain the NaDyF₄:Yb,Er nanoparticles.

### Embodiment 9

Synthesis of LiTmF₄ nanoparticles: adding 1 mmol of lithium trifluoroacetate and 1 mmol of thulium trifluoroacetate into a mixed solvent of oleic acid (20 mmol) and octadecene (20 mmol), heating up to 320°C under the protection of an inert gas, maintaining the temperature for 15 h, cooling the reaction solution to room temperature, adding a large amount of ethanol thereinto, and performing centrifugal washing six times to obtain the LiTmF₄ nanoparticles.

### Embodiment 10

Synthesis of KYb₂F₇ nanoparticles: adding 1 mmol of potassium trifluoroacetate and 1 mmol of ytterbium trifluoroacetate into a mixed solvent of oleic acid (20 mmol) and octadecene (20 mmol), heating up to 310°C under the protection of an inert gas, maintaining the temperature for 2 h, cooling the reaction solution to room temperature, adding a large amount of ethanol thereinto, and performing centrifugal washing six times to obtain the KYb₂F₇ nanoparticles.

### Embodiment 11

Synthesis of BaYF₅ nanoparticles: adding 1 mmol of barium oxalate and 1 mmol of yttrium trifluoroacetate into a mixed solvent of linoleic acid (10 mmol), oleic acid (10 mmol) and octadecylamine (20 mmol), heating up to 340°C under the protection of an inert gas, maintaining the temperature for 24 h, cooling the reaction solution to room temperature, adding a large amount of ethanol thereinto, and performing centrifugal washing six times to obtain the BaYF₅ nanoparticles.

### Embodiment 12

Coating citric acid on particle surfaces: dispersing Gd₂O₃ nanoparticles (0.1 mmol) obtained in Embodiment 1 into 5 ml of chloroform, adding a citric acid aqueous solution (n/n=20), and vigorously stirring at room temperature for at least 6 h; taking the upper suspension liquid, adding a large amount of ethanol and centrifuging, and dispersing the obtained precipitate into pure water to obtain the nanoparticle magnetic resonance contrast agent.

### Embodiment 13

Coating cysteine on particle surfaces: dispersing Y₂O₃ nanoparticles (0.1 mmol) obtained in Embodiment 4 into 5 ml of chloroform, adding a cysteine aqueous solution (n/n=30), and vigorously stirring at room temperature for at least 6 h; taking the upper layer suspension liquid, adding a large amount of ethanol and centrifuging, and dispersing the obtained precipitate into pure water to obtain the nanoparticle magnetic resonance contrast agent.

### Embodiment 14

Coating polyvinyl alcohol on particle surfaces: dispersing CeOF nanoparticles (0.1 mmol) obtained in Embodiment 6 into 10 ml of cyclohexane, adding 10 mL of N,N-dimethyl formamide and 50 mg of nitrosonium tetrafluoroborate, and vigorously stirring at room temperature for no less than 1 h; taking the lower layer liquid, adding a large amount of toluene and centrifuging, dissolving the obtained precipitate into 10 mL of N,N-dimethyl formamide again, adding 50 mg of polyvinyl alcohol, and stirring for no less than 4 h; then adding a large amount of acetone into the solution, centrifuging, and dispersing the obtained precipitate into pure water to obtain the nanoparticle magnetic resonance contrast agent.

### Embodiment 15

Coating polyethylene imine on particle surfaces: dispersing LaF₃ nanoparticles (0.2 mmol) obtained in Embodiment 5 into 10 ml of cyclohexane, adding 10 mL of N,N-dimethyl formamide and 50 mg of nitrosonium tetrafluoroborate, and vigorously stirring for no less than 1 h; taking the lower layer liquid, adding a large amount of toluene and centrifuging, dissolving the obtained precipitate into 10 mL of N,N-dimethyl formamide again, adding 50 mg of polyethylene imine, and stirring for no less than 4 h; then adding a large amount of acetone into the solution, centrifuging, and dispersing the obtained precipitate into pure water to obtain the nanoparticle magnetic resonance contrast agent.

### Embodiment 16

Coating polyethylene pyrrolidinone on particle surfaces: dispersing NaDyF₄:Yb,Er nanoparticles (0.2 mmol) obtained in Embodiment 8 into 10 ml of cyclohexane, adding 10 mL of N,N-dimethyl formamide and 50 mg of nitrosonium tetrafluoroborate, and vigorously stirring for no less than 1 h; taking the lower layer liquid, adding a large amount of toluene and centrifuging, dissolving the obtained precipitate into 10 mL of N,N-dimethyl formamide again, adding 50 mg of polyethylene pyrrolidinone, and stirring for no less than 4 h; then adding a large amount of acetone into the solution, centrifuging, and dispersing the obtained precipitate into pure water to obtain the nanoparticle magnetic resonance contrast agent.

Fig. 1 to Fig. 4 show contrasts of magnetic resonance images obtained by using the rare earth-based nanoparticle magnetic resonance contrast agent obtained from Embodiment 12 and five clinically commonly-used contrast agents under different concentrations, wherein the used magnetic field strengths are 3 T. The used scanning sequence in Fig. 1 is a T₁ weighted sequence; the used scanning sequence in Fig. 2 is a T₂ weighted sequence; the used scanning sequence in Fig. 3 is a ceMRA sequence; and the used scanning sequence in Fig. 4 is a LAVA sequence. It can be seen from Fig. 1 to Fig. 4 that the imaging effect of the rare earth-based nanoparticle magnetic resonance contrast agent obtained in Embodiment 12 is superior to that obtained by using the clinically commonly-used contrast agents under the same concentration, and the contrasting effect is remarkably improved with the increase of the concentration (the brighter images in Fig. 1, Fig. 3, and Fig. 4 indicate a better contrasting effect, and the darker image in Fig. 2 indicates a better contrasting effect). It should be noted that, in Fig. 1 the images of the rare earth-based nanoparticle magnetic resonance contrast agent becomes darkened under a relatively high concentration due to the existence of "saturation effect", that is, at this time the T₁ contrasting effect has reached the limit, and the T₂ contrasting effect will be improved and partially offset the T₁ contrasting effect under a high concentration, which shows that the rare earth-based nanoparticle magnetic resonance contrast agent can achieve the same contrasting effect under a concentration lower than that of the clinically commonly-used contrast agent.

Fig. 5 is a diagram showing a contrast of relaxivities obtained by using the rare earth-based nanoparticle magnetic resonance contrast agent obtained in Embodiment 12 and five clinically commonly-used contrast agents, wherein the used magnetic field strength is 3 T. It can be seen from Fig. 5 that the longitudinal and transverse relaxivities of the rare earth-based nanoparticle magnetic resonance contrast agent obtained in Embodiment 12 are higher than those of the clinically commonly-used contrast agents.

Fig. 6 shows a contrast of a relaxivity obtained by using the rare earth-based nanoparticle magnetic resonance contrast agent obtained in Embodiment 12 at different magnetic field strengths. It can be seen from Fig. 6 that the rare earth-based nanoparticle magnetic resonance contrast agent obtained in Embodiment 12 exhibits high longitudinal and transverse relaxivities at both high magnetic field strength and low magnetic field strength.

The rare earth-based nanoparticle magnetic resonance contrast agent of the present invention can significantly reduce the relaxation time of surrounding protons, thereby greatly increasing the contrast ratio of local tissues. The rare earth-based nanoparticle magnetic resonance contrast agent of the present application has such advantages as high relaxivity, long in vivo residence time, low injection dose, and small leakage possibility of the rare earth ions and the like, and can effectively increase the diagnostic accuracy and the safety of the contrast agent.

The foregoing described embodiments of the present invention are not intended to limit the present invention. Those skilled in the art can make some changes and modifications without departing from the spirit and scope of the invention. Therefore the protective scope of the present invention is defined only by the claims.

## Claims

1. A rare earth-based nanoparticle magnetic resonance contrast agent, **characterized in** being rare earth-based inorganic nanoparticles coated with hydrophilic ligands.

2. The rare earth-based nanoparticle magnetic resonance contrast agent as described in claim 1, **characterized in that** the composition of the rare earth-based nanoparticles is MₐREO_{b}X_{c}, wherein RE represents a rare earth element, M represents an alkali or alkaline earth metal, X represents a fluorine or chlorine, 0≤a≤1, 0≤b≤1.5, and 0≤c≤4; or the rare earth-based inorganic nanoparticles are an inorganic compound doped by using the MₐREO_{b}X_{c} as a substrate.

3. The rare earth-based nanoparticle magnetic resonance contrast agent as described in claim 1, **characterized in that** the rare earth element comprises one or more of lanthanum, cerium, praseodymium, neodymium, samarium, europium, gadolinium, terbium, dysprosium, holmium, erbium, thulium, ytterbium, lutetium, scandium, and yttrium.

4. The rare earth-based nanoparticle magnetic resonance contrast agent as described in claim 1, **characterized in that** the surface coating ligands of the rare earth-based nanoparticles are one or more of the following: citric acid, cysteine, polyvinyl alcohol, polyethyleneimine, polyvinyl pyrrolidone, and polyacrylic acid.

5. A preparation method of the rare earth-based nanoparticle magnetic resonance contrast agent as described in claim 1, **characterized by** comprising the following steps:
1) adding a certain amount of a rare earth precursor or a mixture of a rare earth precursor and a non-rare earth precursor into a high-boiling organic solvent to obtain a solution A;
2) performing vacuum pumping on the solution A to remove moisture, then heating up to 250-340°C under the protection of an inert gas, maintaining for 15 min-24 h, and then cooling to room temperature to obtain a sol B;
3) performing centrifugal separation on the sol B, washing the obtained precipitate, and then coating the surface of the precipitate with hydrophilic ligands; and
4) dispersing the coated particles into a solvent to obtain the contrast agent.

6. The method as described in claim 5, **characterized in that** the high-boiling organic solvent refers to a mixed solvent composed of one or more of oleic acid, linoleic acid, oleylamine, octadecene, hexadecylamine and octadecylamine.

7. The method as described in claim 5, **characterized in that** the rare earth precursor is a mixture of one or more of the following: rare-earth hydroxides, oxalates, acetates, trifluoroacetates, trichloroacetates, acetylacetonates, and phenyl acetylacetonates; and the non-rare earth precursor is a mixture of one or more of the following: alkali-metal and alkaline earth-metal fluorides, hydroxides, oxalates, acetates, trifluoroacetates, trichloroacetates, acetylacetonates, and phenyl acetylacetonates.

8. The method as described in claim 5, **characterized in that** the molar ratio of the precursor to the solvent is 1:20-1:200 in step 1); vacuum pumping is performed at 100-140°C in step 2); and a large amount of ethanol is employed to wash in step 3).

9. The method as described in claim 5, **characterized in that** a washing manner employed in step 3) is centrifugal washing, and washing is performed for 2 to 6 times.

10. The method as described in claim 5, **characterized in that** the solvent is water or physiological saline in step 4).
